# EUROPEAN PATENT APPLICATION

(11) **EP 4 001 921 A1**
(43) Date of publication of application: **25.05.2022**
(21) Application number: 20207596.6
(22) Date of filing: 13.11.2020
(51) Int. Cl.: G01N 33/92

(54) **EXTRACELLULAR VESICLE MARKERS FOR CHRONIC CORONARY SYNDROME**

(71) Applicant: UMC Utrecht Holding B.V., 3584 CM Utrecht (NL)
(72) Inventor: DE KLEIJN, Dominicus Paschalis Victor, 3584CM, Utrecht (NL)
(74) Representative: De Vries & Metman

(57) **Abstract**

The present invention relates to the determination of protein markers associated with extracellular vesicles present in sub-fractions of plasma samples taken from people that experience chest pain and are suspected to experience chronic coronary syndrome (CCS) also known as stable angina. The invention relates to the use of the markers in the identification of subjects suffering from, or at risk of suffering from, CCS. Especially preferred protein markers are CD31, NT-proBNP and Cathepsin D.

## Description

### FIELD OF THE INVENTION

The invention relates to the field of medicine. More in particular it relates to a method for identifying a subject suffering from, or being at risk of suffering from,
chronic coronary syndrome by measuring the concentration or a value related thereto of one or more protein markers in one or more blood plasma fractions. The protein markers are especially suitable for identifying a subject suffering from, or being at risk of suffering from stable angina, and equivalents thereof.

### BACKGROUND ART

### Chronic Coronary Syndrome

Chronic coronary syndrome (CCS) has been defined as chronic stable coronary artery disease, either a-symptomatic or symptomatic, but excludes the clinical scenario when an acute coronary artery thrombosis dominates the clinical presentation which is referred to a Acute Coronary Syndrome (ACS).

Stable Coronary Artery Disease is the underlying cause of the clinical syndrome referred to as 'stable angina': episodes of stress-, exercise- or emotion-induced chest pain. Stable coronary artery disease can also cause atypical symptoms such as shortness of breath or reduced exercise tolerance. These symptoms are not generally referred to as stable angina, but can be referred to as 'stable angina equivalents' as they are caused by the same underlying disorder. Stable coronary artery disease is generally characterized by episodes of reversible myocardial demand/supply mismatch due to significant stenosis in the coronary arteries. Because the appearance of chest pain or equivalent symptoms are in a sense predictable, the disease was referred to as *stable* angina. The terms 'stable angina' and 'stable coronary artery disease' are often mixed and often used in the art to mean the same thing and have now, in the 2019 guidelines, been replaced by the term 'Chronic coronary syndrome" (hereinafter referred to as CCS). (Knuuti et al, 2019 ESC Guidelines for the diagnosis and management of chronic coronary syndromes Eur. Heart J. 2020, 41, 404-477*).*
For clarity reasons, 'stable angina' as used herein refers to all symptoms (predictable episodes of stress-, exercise- or emotion-induced chest pain, and atypical symptoms such as shortness of breath or reduced exercise tolerance) that are caused by CCS, formerly known as stable coronary artery disease. In the context of this disclosure, the terms stable angina, stable coronary artery disease and CCS are used interchangeably to address the same clinical indication.
The prevalence of stable angina varies from 5-14% depending on gender and age. Annual incidence of death is 1.2-2.4% (vs 0.6% in subjects without obstructive coronary artery disease), and the annual incidence of myocardial infarction is 2.7%. Because of the variety in clinical presentation and a broad differential diagnosis (such as musculoskeletal or psychological problems, pulmonary embolism, pneumonia, pneumothorax, pericarditis), the diagnosis of stable angina is notoriously challenging. Patients can be referred for exercise ECG or non-invasive imaging such as cardiac CT, MRI or nuclear scan. Such imaging tests have a sensitivity and specificity of around 85%, and are time consuming, include exposure to radiation and are expensive. In many cases, invasive coronary angiogram is needed to confirm the diagnosis and to determine the options for revascularisation by percutaneous coronary intervention or coronary artery bypass grafting. The diagnostic work-up is inefficient and expensive. A rapid straightforward test for diagnosing (or ruling out) stable angina or CCS is currently non-existent.

### Blood biomarkers

Clearly, as outlined above, the establishment of a timely identification of a subject suffering from, or being at risk of suffering from, CCS is important to allow adequate treatment. An accurate early diagnosis may take away the symptoms and improve the prognosis of the patients. In addition, ruling out both diseases prevents unnecessary referrals and hospital admissions for time consuming non-invasive and invasive diagnostic testing, thereby significantly decreasing the health care burden. A simple and rapid test for all patients presenting with symptoms suggestive of CCS is highly desired. It has been recognized in the art that a blood test is the most convenient, since it is a low risk diagnostic tool and easily accessible in both primary and secondary care.
Many proteins have been investigated in relation to stable angina. The inflammatory concept of atherosclerosis led many investigators to concentrate on inflammatory mediators such as hsCRP, GDF-15, neopterin, IL-6, IL-10, IL-17, MPO, procalcitonin, Fetuin A, Lp-PLA2, and MMPs/TIMPs. Although these markers have some prognostic value, none of them appear to have sufficient diagnostic power to discriminate patients with chest pain due to stable angina (CCS) from those that have chest pain due to non-cardial causes. (Tsaknis et al. Clinical usefulness of novel serum and imaging biomarkers in risk stratification of patients with stable angina. Dis Markers 2014:831364).

Also non-protein markers have been investigated. In a small pilot study involving 53 patients, three micro RNAs (miRNAs) appeared to have the potential to discriminate patients with stable angina from controls. MiRNAs are small non-coding RNAs that regulate complex biological processes. The miRNAs that were found were miR-1, miR-126, miR-483-5p, having an Area Under the Curve (AUC) of 0.91, 0.92 and 0.85 respectively (D'Alessandra et al. Diagnostic potential of plasmatic MicroRNA signatures in stable and unstable angina. PLoS One. 2013 Nov 15:8). However, these miRNAs have thus far not been validated. It is evident that miRNAs could potentially be used as biomarkers for stable angina, but additional studies are required to validate their potential and to address inconsistencies between the different studies. Next to this, detection of miRNA is technically challenging requiring cDNA synthesis and qPCR which limits their application in an acute setting or in GP settings.

It is concluded that there remains an urgent need for alternative circulating biomarkers to identify subjects suffering from, or being at risk of suffering from, chronic coronary syndrome (CCS) and to discriminate between life-threatening events such as stable angina of patients with chest pain or equivalent symptoms on the one hand, and milder events in people that also experience similar symptoms but do not suffer from such chronic coronary disease, on the other hand.

### SUMMARY OF THE INVENTION

The present invention relates to a method for identifying a subject suffering from, or being at risk of suffering from, chronic coronary syndrome, said method comprising the steps of:
a) obtaining one or more of the plasma fractions selected from the group consisting of the Low-Density Lipoprotein (LDL) fraction and the High-Density Lipoprotein (HDL) fraction from a plasma sample of said subject;
b) determining one or more values, wherein each of the one or more values:
   - is derived from a concentration of a protein marker in one of said plasma fractions, wherein the protein marker is selected from the group consisting of CD31, NT-proBNP and Cathepsin D, and/or
c) is a ratio of two values derived from the concentrations of a single protein marker in two different ones of said plasma fractions, wherein the protein marker is selected from the group consisting of CD31, NT-proBNP and Cathepsin D,
performing a comparison of the one or more values as determined in step b) with one or more corresponding reference values, which has been derived in the same way from the concentration of the same one or more protein marker in corresponding plasma fractions as determined in a group of reference subjects not suffering from CCS, wherein a statistically significant difference between the one or more values determined in step b) and the one or more corresponding reference values is indicative of the subject suffering, or being at risk of suffering from, CCS.
By this method it is for the first time possible to identify subjects suffering from chronic coronary syndrome, also known as stable angina, from a blood sample with a high level of statistical significance.

In a preferred embodiment, the
at least one of the one or more values in step b) is a value selected from the group consisting of:
- a value derived from the concentration of NT-proBNP in the HDL plasma fraction
- a value derived from the concentration of NT-proBNP in the LDL plasma fraction,
- a value derived from the concentration of CD31 in the LDL plasma fraction, and
- a value derived from the concentration of Cathepsin D in the LDL plasma fraction;
or a ratio a value selected from the group consisting of;
- the ratio of the value derived from the concentration of NT-proBNP in the LDL plasma fraction over the value derived from the concentration in the HDL plasma fraction,
- the ratio of the value derived from the concentration of CD31 in the LDL plasma fraction over the value derived from the concentration in the HDL plasma fraction, and
- the ratio of the value derived from the concentration of CTSD in the LDL plasma fraction over the value derived from the concentration in the HDL plasma fraction.
In another preferred embodiment step b) involves determination of at least two values involving a protein marker selected from the group consisting of NT-proBNP, CD31 and Cathepsin D, more in particular step b) involves determination of at least two values involving two different protein markers selected from the group consisting of NT-proBNP, CD31 and Cathepsin D. This embodiment of the invention provides for the first time a method to identify subjects suffering from, or being at risk of suffering from, CCS from a blood sample with a high level of statistical significance.

In a further embodiment, the present invention relates to a kit comprising means for performing the method according to the invention.

### BRIEF DESCRIPTION OF THE DRAWINGS

These and other aspects of the invention are apparent from and will be elucidated with reference to the embodiments described hereinafter.

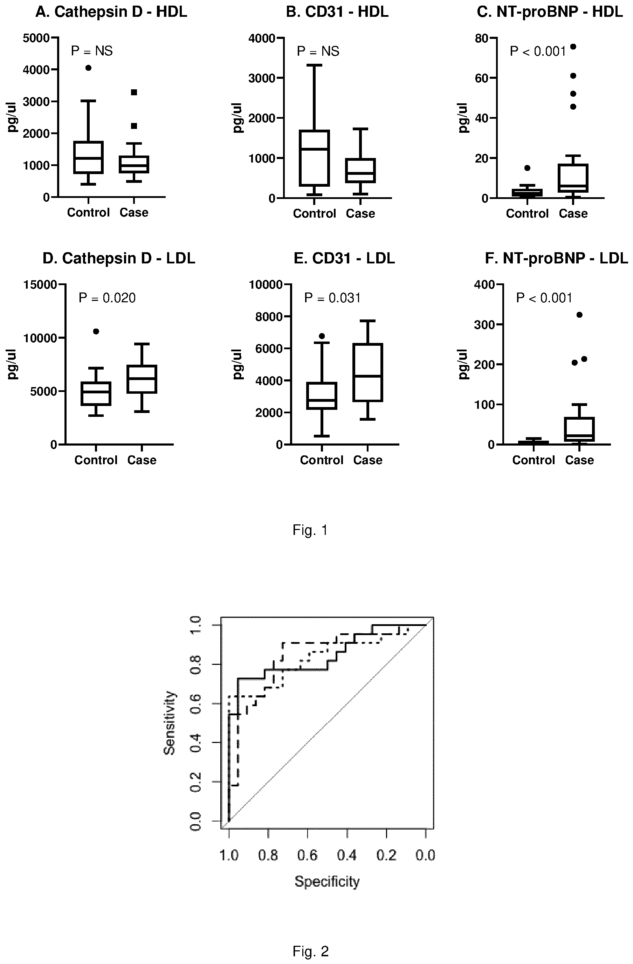

**Figure 1** Verification of three selected proteins in the 22 Cases with SA and 22 matched chest pain controls using Mesoscale Diagnostics (MSD) immuno assays. Figure 1A-C shows boxplots with MSD results for the selected three proteins (Cathepsin D, CD31, and NT-proBNP) in the HDL subfraction. In figure 1D-F the results for the LDL subfraction are summarized.
**Figure 2** Area Under the Curve (AUC) graphs of the best extracellular vesicle protein or combinations of extracellular vesicle proteins in the two plasma fractions LDL and HDL. Solid black line is the marker/fraction pair combination CatDHDL_BNPLDL, AUC: 0.847 (0.728-0.966); Sensitivity: 72.7%; Specificity: 90.9%; PPV 94.1%; NPV 77.8%. Dashed line is the marker/fraction pair combination CD31HDL_BNPLDL, AUC: 0.845 (0.725-0.965); Sensitivity: 90.9%; Specificity: 72.7%; PPV 76.9%; NPV 88.9%. Dotted line is the single marker/fraction pair BNP_LDL, AUC: 0.833 (0.708-0.958); Sensitivity: 77.3%; Specificity: 72.7%; PPV 73.9%; NPV 76.2%

### DETAILED DESCRIPTION OF EMBODIMENTS

The present invention relates to a method for identifying a subject suffering from, or being at risk of suffering from, chronic coronary syndrome (CCS) from a blood sample based on the concentration (or a value derived from the concentration) of one or more identified protein markers in one or more blood plasma fractions or based on the ratio of concentration (or values derived from the concentration) of a given protein marker in two different ones of said plasma fractions and comparing it to the corresponding concentration and/or ratio as determined in a group of reference subjects not suffering from CCS, wherein a statistically significant difference between the concentration and/or ratio determined for the subject in question and the corresponding concentration and/or ratio of the reference group is indicative of the subject suffering, or being at risk of suffering, from CCS. In the context of the present invention, "concentration" of a given protein marker in a given plasma sub-fraction can also mean a "value derived from the concentration" of the protein marker in the plasma sub-fraction in question, wherein a value derived from the concentration is a value that directly correlates with the concentration of the protein marker.
The present invention provides for a method for identifying a subject suffering from, or being at risk of suffering from, CCS, said method comprising the steps of:
a) obtaining one or more of the plasma fractions selected from the group consisting of the Low-Density Lipoprotein (LDL) fraction and the High-Density Lipoprotein (HDL) fraction from a plasma sample of said subject;
b) determining one or more values, wherein each of the one or more values:
   - is derived from a concentration of a protein marker in one of said plasma fractions, wherein the protein marker is selected from the group consisting of CD31, NT-proBNP and Cathepsin D;
   and/or
   - is a ratio of two values derived from the concentrations of a single protein marker in two different ones of said plasma fractions, wherein the protein marker is selected from the group consisting of CD31, NT-proBNP and Cathepsin D;
c) performing a comparison of the one or more values as determined in step b) with one or more corresponding reference values, which has been derived in the same way from the concentration of the same one or more protein marker in corresponding plasma fractions as determined in a group of reference subjects not suffering from CCS, wherein a statistically significant difference between the one or more values determined in step b) and the one or more corresponding reference values is indicative of the subject suffering, or being at risk of suffering, from CCS.
By this method it is for the first time possible to identify subjects suffering from CCS, also known as stable angina, from a blood sample with a statistical relevance characterized by that the one or more values in step b) are selected such that the area under the curve (AUC) differs from the diagonal reference line of 0.5 with a p-value of 0.05 or less, as determined by Receiver Operating Characteristic (ROC) plot analysis of the combination of said one or more values based on a suitable group of definitive subjects and group of reference subjects. Preferably, the statistical relevance of the method according to the invention is characterized by that the one or more values in step b) are selected such that the area under the curve (AUC) is 0.8 or more, such as 0.85 or more, 0.9 or more or 0.95 or more and the p-value is 0.05 or less, as determined by Receiver Operating Characteristic (ROC) plot analysis of the combination of said one or more values based on a suitable group of definitive subjects and group of reference subjects. Even more preferably, the statistical relevance of the method according to the invention is characterized by resulting in a negative predictive value and/or a positive predictive value is 0.8 or more, such as 0.85 or more, 0.9 or more or 0.95 or more. In one embodiment, the one or more value is a value determined from a single protein marker selected from the group of CD31, Cathepsin D or NT-proBNP. In another embodiment, the one or more values are two values determined from two different protein markers selected from the group of CD31, Cathepsin D or NT-proBNP. In yet another embodiment, the protein marker preferably is NT-proBNP. The method according to the present invention is an in vitro method.

In the context of the present invention, the subject may be any mammal but is preferably a human subject and even more preferably a human patient, such as a human patient having chest pain and/or dyspnea. In the context of the present invention, the group of reference subjects is of the same origin as the subject itself, and accordingly, if the subject is a human, the group of reference subjects are also human. In one embodiment of the present invention, the group of reference subjects have the same clinical signs and symptoms as the subject itself but are not suffering from CCS.

The plasma fractions used in the present invention can be obtained as follows:
Using dextrane sulphate and Mn, chylomicrons, Very Low-Density Lipoprotein (VLDL) and Low-Density Lipoprotein (LDL) and plasma Extracellular Vesicles are precipitated in the first fractionation step. Hereinafter, the plasma fraction obtained in this first fractionation step is called "LDL". In the second step High-Density Lipoprotein (HDL) is precipitated. Sub-fractionation of the LDL and HDL fractions from plasma is known in the art. The inventors of the present invention have investigated the protein content of plasma extracellular vesicles present in the blood plasma sub-fractions LDL and HDL. In the context of the present invention, the term "plasma fraction" refers to the LDL and HDL plasma fractions. The terms "plasma fraction" and "plasma sub-fraction" are used interchangeably herein.

Plasma extracellular vesicles are bilayer lipid membrane vesicles including exosomes, microvesicles and microparticles (Colombo et al. Biogenesis, secretion, and intercellular interactions of exosomes and other extracellular vesicles. Ann Rev Cell Dev Biol 2014:255-289). Exosomes are synthesized in the multivesicular endosome, while microvesicles are formed by the plasma membrane. Once secreted in the plasma these extracellular vesicles can no longer be distinguished from each other. This is why exosomes are often called microvesicles and microvesicles are often referred to as exosomes. For the sake of clarity, extracellular vesicles as used herein refer to all such extracellular bilayer lipid membrane vesicles present in the sub-fractions of the plasma, as outlined further below.
Extracellular vesicles play an important role in intercellular communication and contain or are associated with proteins, miRNAs and mRNA from the cell of origin, reflecting their physiological or pathological status. It is known that distinct bilayer membrane extracellular plasma vesicles co-fractionate with monolayer LDL. Other bilayer membrane extracellular vesicles (with a different content) co-fractionate with HDL (Zhang et al. Circulating TNFR1 exosome-like vesicles partition with the LDL fraction of human plasma. Biochem Biophys Res Comm 2008:579-584*).* This allows separation of distinct plasma extracellular vesicle sub-fractions via sequential LDL and HDL isolation. Through this, the inventors were able to identify subpopulations of extracellular vesicles, each with their own particular protein content and potentially (patho)-physiological pathways associated therewith. Plasma extracellular vesicles have been recognized in the art as having potential value in relation to cardiovascular disease (Wang et al. Plasma extracellular vesicle protein content for diagnosis and prognosis of global cardiovascular disease. Neth Heart J 2013:467-471*).*

In the context of the present invention, protein marker NT-proBNP is identified as the polypeptide corresponding to amino acids 27-103 of the full length *UniProtKB* - *P16860 (ANFB_HUMAN)* protein, CD31, also known as PECAM-1, is identified as the protein with the amino acid sequence depicted in *UniProtKB* - *P16284 (PECA1_HUMAN),* and Cathepsin D is identified as the protein with the amino acid sequence depicted in *UniProtKB* - *P07339 (CATD_HUMAN),* when the subject is a human, such as a human patient. The skilled person will understand, that if the subject is another mammal than human, the protein markers to be used in accordance with the invention, will be the corresponding proteins in the mammal in question.

In the context of the present invention, a value derived from the concentration of a given protein marker in a given plasma fraction is also called a value of a "marker/fraction pair". For convenience, a marker/fraction pair is sometimes abbreviated herein by [name of the marker]-[name of the plasma fraction]. For example, the protein marker NT-proBNP determined in plasma fraction LDL interchangeably is also referred to by "NT-proBNP in LDL", "NT-proBNP-LDL" or "BNP-LDL". Likewise, a value which is a ratio of two values derived from the concentrations of a given protein marker in two different plasma fractions is called a value of a "marker/ratio pair". For convenience, a marker/ratio pair is sometimes abbreviated herein by [name of the marker]-[name of plasma fraction 1]/[name of plasma fraction 2]. For example, the protein marker NT-proBNP determined in plasma fraction LDL and HDL and for which the ratio of the values derived from the concentrations in LDL over HDL is used, is also referred to by "ratio of NT-proBNP in LDL over HDL", "NT-proBNP-LDL/HDL" or "BNP-LDL/HDL". In the context of the present invention, it was found that for the marker/ratio pair it is irrelevant whether the LDL or HDL plasma fraction was the plasma fraction 1 since the ratio of the two values resulted in the same Area Under the Curve (AUC) and significance (p-value) in Receiver Operating Characteristic analysis and statistical tests. Thus [name of marker]-LDL/HDL refers to the same ratio as [name of marker]-HDL/LDL and the abbreviations can be used interchangeably. For convenience the abbreviation [name of marker]-LDL/HDL is used when referring to the ratio of two values derived from the concentrations of a given protein marker in the LDL and HDL plasma fractions.
The value derived from the concentration of a given protein marker in a given plasma fraction can be determined in any way known to a person skilled in the art. In one embodiment of the present invention, the determination of the one or more values in step b) is performed by an immunoassay using antibodies specific to the one or more protein markers in question. The immunoassay can suitably be a beads-based immunoassay, wherein the beads are conjugated with the selected antibodies to synthesize the bead-capture antibody complex. The bead-capture antibody complex is then incubated with the samples and subsequently with biotinylated antibodies to detect the captured protein by reaction with streptavidin subsequent and quantification.

The one or more values of marker/fraction and/or marker/ratio pairs selected in step b), are selected based on their individual or combined statistical relevance for identification of a subject suffering from, or at risk of suffering from CCS. The statistical relevance can be determined in any way known to the person skilled in the art. Logistic regression analysis is often used to predict a binary outcome (yes or no). In medical research it is often used to predict if a patient has a certain disease, for example diabetes (yes or no) by modelling observed characteristics of the patients e.g. sex, age, weight and systolic blood pressure. In the diabetes example a result from logistic regression could be that a 10-year increase in age gives 20% higher odds of having diabetes. By combining more probabilities, based on more than one patient characteristic in one prediction model, one can even more accurately predict if a patient has diabetes or not. Next to using patient characteristics like sex and age, logistic regression can also be performed with biomarker levels associated with extracellular vesicles as potential predictors. A set of biomarkers can be combined in one model and used to estimate the probability of a disease. The performance of a logistic regression model can be visualized in a Receiver Operating Characteristic (ROC) plot. The higher the Area Under the Curve (AUC), the better the performance of the model. An AUC of 0.5 corresponds to a 50% chance of the disease in question (flipping a coin). Here, the inventors used logistic regression and ROC plot analysis in order to evaluate whether the diagnosis of CCS (or stable angina or stable coronary artery disease) could be improved based on the three extracellular vesicle proteins in 2 different plasma sub-fractions and whether such be better than by random chance. The present inventors determined the statistical relevance of the different individual marker/fraction pairs and marker/ratio pairs and of combinations thereof by ROC plot analysis. The ROC plot analysis involves:
- analyzing Differences in baseline-characteristics using Chi-square test for categorical variables
- performing T-tests for normally distributed continuous variables and Mann-Whitney-U-tests for continuous variables that were not normally distributed
- converting the one or more values derived from the concentration in a given plasma fraction or ratio of concentrations in two different plasma fractions into standard deviation units, or the z-score, by using the observed value minus the mean value, divided by the standard deviation, and
- Performing Receiver-operating characteristic (ROC) analysis (ROC plot) to determine the area under the curve (AUC) and optimal calculated cutoff for the negative predictive value (NPV) and/or the positive predictive value (PPV).
This statistical analysis can be performed by a any suitable software, for example by SPSS^{®} (IBM^{®}, Version 22) and Rstudio using R software for statistical computing version 3.1.2. The skilled person will understand, that where more than one value of marker/fraction and/or marker/ratio pairs are used, the AUC and calculated cut-off value obtained from the ROC plot analysis relate to the combination of said values.

In one embodiment of the present invention the one or more values in step b) are selected such that the area under the curve (AUC) differs from the diagonal reference line of 0.5 with a p-value of 0.05 or less, as determined by Receiver Operating Characteristic (ROC) plot analysis of the combination of said one or more values based on a suitable group of definitive subjects and group of reference subjects. In a further embodiment, the one or more values in step b) are selected such that the area under the curve (AUC) is 0.8 or more, such as 0.85 or more, 0.9 or more or 0.95 or more, and the p-value is 0.05 or less, as determined by Receiver Operating Characteristic (ROC) plot analysis of the combination of said one or more values based on a suitable group of definitive subjects and group of reference subjects. In yet a further embodiment, the negative predictive value and/or the positive predictive value is 0.8 or more, such as 0.85 or more, 0.9 or more or 0.95 or more, for the selected one or more values in step b), when using the optimal cut-off value as determined by Receiver Operating Characteristic (ROC) plot analysis of the combination of said one or more values based on a suitable group of definitive subjects and group of reference subjects. In the context of the present invention the term "group of definitive subjects" means a group of subjects that has been verified to suffer from CCS by other means than by the method of the present invention. This group could also be called the positive control group. The term "group of reference subjects" as used herein, is a group of subjects that has been verified not to suffer from CCS by other means than by the method of the present invention. This group could also be called the negative control group. In one embodiment of the present invention, the group of reference subjects have the same clinical signs and symptoms as the group of definitive cases but are not suffering from CCS. In a further embodiment, the suitable group of definitive subjects and group of reference subjects is a suitable cohort, such as a cohort selected from the group consisting of the Myomarker cohort. In the context of the present invention, the Myomarker cohort comprises a group of definitive subjects, which have been diagnosed to suffer from CCS and a group of reference subjects, which have the same clinical signs and symptoms as the group of definitive cases, but which are not suffering from CCS, wherein the assessment of whether or not a subject falls in the group of definitive cases or in the group of reference subjects is performed by other means than by the method of the present invention. The comparison performed in step c) of the method according to the present invention can therefore alternatively be implemented as performing a comparison using a model and a cut-off value as determined by Receiver Operating Characteristic (ROC) plot analysis of the combination of said one or more values based on a suitable group of definitive subjects and group of reference subjects, wherein the outcome of said model is indicative of the subject suffering from, or being at risk of suffering from, CCS.

In a further embodiment, the present invention relates to a method for determining one or more values derived from a concentration of a protein marker in a plasma fraction or from a ratio of concentrations of a single protein marker in two different plasma fractions, said method comprising the steps of:
a) obtaining one or more of the plasma fractions selected from the group consisting of the Low-Density Lipoprotein (LDL) fraction and the High-Density Lipoprotein (HDL) fraction from a plasma sample of said subject;
b) determining one or more values, wherein each of the one or more values:
   - is derived from a concentration of a protein marker in one of said plasma fractions, wherein the protein marker is selected from the group consisting of CD31, NT-proBNP and Cathepsin D,
   and/or
   - is a ratio of two values derived from the concentrations of a single protein marker in two different ones of said plasma fractions, wherein the protein marker is selected from the group consisting of CD31, NT-proBNP and Cathepsin D.
The method may further comprise performing a comparison of the one or more values as determined in step b) with one or more corresponding reference values, which has been derived in the same way from the concentration of the same one or more protein marker in corresponding plasma fractions as determined in a group of reference subjects not suffering from CCS. When there is a statistically significant difference between the one or more values determined in step b) and the one or more corresponding reference values, it is indicative of the subject suffering, or being at risk of suffering, from CCS.

In another embodiment, the present invention relates to a method for determining one or more values derived from a concentration of a protein marker in a plasma fraction or from a ratio of concentrations of a single protein marker in two different plasma fractions, wherein each of the one or more values
- is selected from the group consisting of a value derived from the concentration of CD31 in the LDL plasma fraction, a value derived from the concentration of NT-proBNP in the LDL plasma fraction, a value derived from the concentration of NT-proBNP in the HDL plasma fraction, and a value derived from the concentration of Cathepsin D in the LDL plasma fraction,
and/or
- is a ratio selected from the group consisting of the ratio of the value derived from the concentration of CD31 in the LDL plasma fraction over the value derived from the concentration in the HDL plasma fraction, the ratio of the value derived from the concentration of NT-proBNP in the LDL plasma fraction over the value derived from the concentration in the HDL plasma fraction, and the ratio of the value derived from the concentration of CTSD in the LDL plasma fraction over the value derived from the concentration in the HDL plasma fraction.
The present inventors have found that these marker/fraction pairs and marker/ratio pairs individually provide a statistically relevant identification of subjects suffering from CCS on their own (see Table 3). Accordingly, combination with further marker/fraction and/or marker/ratio values is not required but will in many cases improve the certainty by which a subject is correctly identified as suffering from stable angina.

The present inventors also have found that in particular the marker/fraction pairs NT-proBNP in LDL fraction (BNP-LDL) and NT-proBNP in the HDL plasma fraction (BNP-HDL) and the marker/ratio pair ratio of the two values of the NT-proBNP concentration in the LDL plasma fraction over the concentration in the HDL plasma fraction (BNP-LDL/HDL), individually provide AUC values of 0.8 or more, which makes these pairs the most significant individual marker/fraction and marker/ratio pairs for identification of stable angina on their own (without combination with a further marker/fraction or marker/ratio value). Thus, NT-proBNP is a preferred protein marker to identify a patient suffering from, or at risk of suffering from, CCS.
Accordingly, in preferred embodiment of the method for identifying a subject suffering from, or being at risk of suffering from, CCS, at least one of the one or more values is a value derived from the concentration of NT-pro-BNP in the LDL plasma fraction, a value derived from the concentration of NT-pro-BNP in the HDL plasma fraction and/or the ratio of the value derived from the concentration of NT-proBNP in the LDL plasma fraction over the value derived from the concentration in the HDL plasma fraction. Most preferred is the concentration of NT-pro-BNP in the LDL plasma fraction.

### Combination of marker/fraction and/or marker/ratio pairs.

The present inventors also have found that combinations of a marker/fraction pair or marker/ratio pair with a further marker/fraction pair or marker/ratio pairs in many cases improve the certainty by which a subject is correctly identified as suffering from, or at risk of suffering from, CCS . Thus, in another embodiment of the method for identifying a subject suffering from, or being at risk of suffering from, CCS, step b) involves determining at least two values, wherein each of the at least two values
- is derived from a concentration of a protein marker in one of said plasma fractions, and/or
- is a ratio of two values derived from the concentrations of a single protein marker in two different ones of said plasma fractions,
wherein the protein marker is selected from the group consisting of CD31, NT-proBNP and Cathepsin D. Statistically relevant identification of subjects suffering from, or st risk of suffering from, CCS have been obtained when the at least two values are derived from the same protein marker, preferably NT-proBNP. A particularly preferred combination is the combination of the marker/fraction pair BNP-LDL with the marker/ratio pair BNP-LDL/HDL.

Even better statistically relevant identification of subjects suffering from, or at risk of suffereing from, CCS have been obtained when the at least two values are derived from different proteins. Thus, in yet another prefered embodiment of the method for identifying a subject suffering from, or being at risk of suffering from, CCS, step b) of the method comprises determining at least two values involving two different protein markers selected from the group of CD31, NT-proBNP and/or Cathepsin D. The most significant and best performing combinations of a marker/fraction pair or marker/ratio pair with a further marker/fraction pair or marker/ratio pairs are those combinations wherein at least one of the protein markers is NT-proBNP, and the further protein marker is selected from CD31 and Cathepsin D.

The inventors have found that the statistically most significant combination of two marker/fractions and/or marker/ratio pairs for identification of stable angina are
the combination of the concentration of Cathepsin D in the HDL fraction with the concentration of NT-proBNP in LDL fraction;
the combination of the concentration of CD31 in the HDL plasma fraction with the concentration of NT-proBNP in LDL fraction;
the combination of the concentration of Cathepsin D in the HDL fraction with the concentration of NT-proBNP in HDL fraction;
the combination of the concentration of CD31 in the HDL fraction with the concentration of NT-proBNP in HDL fraction;
the combination of the concentration of Cathepsin D in the LDL plasma fraction with the concentration of NT-proBNP in the LDL plasma fraction;
the combination of the concentration of CD31 in the LDL plasma fraction with the concentration of NT-proBNP in the LDL plasma fraction;
the combination of the ratio of the concentration of Cathepsin D in the LDL over HDL plasma fraction with the concentration of NT-proBNP in the LDL plasma fraction;
the combination of the concentration of Cathepsin D in LDL plasma fraction with the concentration of NT-proBNP in the LDL plasma fraction;
the combination of the concentration of Cathepsin D in the HDL plasma fraction with the ratio of the CD31 concentrations in the LDL and HDL plasma fractions;
the combination of concentration of Cathepsin D in the HDL plasma fraction with the concentration of CD31 in the LDL plasma fraction;
the combination of the ratio of the CD31 concentration in the LDL and HDL plasma fractions with the concentration of the NT-proBNP in the LDL plasma fraction; and
the combination of the concentration of CD31 in the LDL plasma fraction with the concentration of the NT-proBNP in the HDL plasma fraction.
Preferred combinations are the combination of the concentration of Cathepsin D in the HDL fraction with the concentration of NT-proBNP in LDL fraction; the combination of the concentration of CD31 in the HDL plasma fraction with the concentration of NT-proBNP in LDL fraction; the combination of the concentration of Cathepsin D in the HDL fraction with the concentration of NT-proBNP in HDL fraction; the combination of the concentration of CD31 in the HDL plasma fraction with the concentration of NT-proBNP in the HDL plasma fraction; and the combination of the concentration of Cathepsin D in the LDL plasma fraction with the concentration of NT-proBNP in the LDL plasma fraction;, more in particular the combination of the concentration of Cathepsin D in the HDL fraction with the concentration of NT-proBNP in LDL fraction; the combination of the concentration of CD31 in the HDL plasma fraction with the concentration of NT-proBNP in LDL fraction; and the combination of the concentration of Cathepsin D in the HDL fraction with the concentration of NT-proBNP in HDL fraction, and the combination of the concentration of CD31 in the HDL plasma fraction with the concentration of NT-proBNP in HDL fraction.
The combination of the concentration of Cathepsin D in the HDL fraction with the concentration of NT-proBNP in LDL fraction and the combination of the concentration of CD31 in the HDL plasma fraction with the concentration of NT-proBNP in LDL fraction are highly preferred combinations, because these are the most significant and best performing combinations of the method of the invention.

Thus, the present invention provides a method and means to discriminate between patients that experience CCS and should be further treated, from patients that also have chest pain, but that do not suffer from CCS, by preferably applying a protein concentration determination of CD31, NT-proBNP and/or Cathepsin D in the LDL and HDL plasma fraction, and comparing the concentrations with those found in a control sample. Although applying a different cohort and patient group, it is concluded that blood-based assay as outlined above can determine whether patients suffer from CCS or not.

In conclusion, the skilled person will understand, that whereas the marker/fraction and marker/ratio pairs identified as having a statistically significant predictive value for the identification of subjects suffering from, or at risk of suffering from, CCS, the predictive value will increase if more than one, such as at least two or at least three of the identified marker/fraction or marker/ratio pairs are included in the methods according to the present invention. In one embodiment of the present invention, the method for identifying a subject suffering from, or being at risk of suffering from, CCS, comprises that at least two values are determined in step b). In another embodiment at least two values are determined in step b) and at least one of these values is derived from concentrations of NT-proBNP.
In yet another embodiment of the present invention, at least two values involving at least two different protein markers are determined in step b), which values each can be selected from a value of a marker/fraction pair or a marker/ratio pair.
In yet a further embodiment of the present invention at least one of said protein markers is NT-proBNP, meaning that either a marker/fraction pair or a marker/ratio pair involving NT-proBNP is used in the method for identifying a subject suffering from, or being at risk of suffering from, CCS.

### Kit

The present invention also relates to a kit comprising means for performing the method according to the invention. In one embodiment, the kit according to the invention comprises means for determining the concentration of said protein markers in said fractions. Said means may comprise any suitable means known in the art, such as an immune-bead based process as outlined herein. Further, the kit may comprise instructions and means for the fractionation of a plasma sample to enable the rapid fractionation and subsequent protein concentration determination as outlined herein. The kit may also comprise means for performing the comparison of step c) in the form of an algorithm with a suitable cut-off value. Preferably, the cut-off value is determined by Receiver Operating Characteristic (ROC) plot analysis of the combination of said one or more values based on a suitable group of definitive subjects and group of reference subjects.

Patients with suspected CCS constitute an enormous health care burden. Rapid and straightforward diagnostic tools for stable angina are currently not available. The technology used in this invention to characterize the protein content of the extracellular vesicles of the different plasma sub-fractions and the subsequent immune-based detection of the indicated proteins in such fractions is suitable for automated settings and ideal for point of care applications, and has major implications for the efficiency of the diagnostic process and clinical management. The diagnosis of CCS with the test as indicated herein now enables timely and adequate subsequent treatment. It will have a significant impact on the prognosis of the patient. On the other hand, ruling out both diseases prevents unnecessary hospital referrals/admissions that are expensive, time consuming and sometimes contain risky additional testing.

To conclude, the inventors of the present invention have found that particular proteins are differentially expressed between sub-fractions of plasma samples of human patients with CCS and reference sub-fractions of plasma samples from human subjects that do not suffer from CCS. Reference samples means that the plasma samples are treated in the same manner and sub-fractions LDL and HDL are compared. Preferably, to have a proper comparison between patient and non-patient (yes/no suffering from CCS) said human patient and said human subject not suffering from CCS both experience chest pain, indicative of CCS. However, the human subject not suffering from CCS may have entered the emergency room or GP office with chest pain that appeared indicative of CCS. Of course the skilled person would understand that other reference samples may also be used in the comparison, for instance those that are obtained from human subjects that are healthy and did not experience chest pain or other symptoms that are related, or that are indicative of CCS.
As outlined above, no rapid and reliable protein-based markers are available in the art that enable a physician or GP to determine quickly whether a human patient suffering from symptoms that are indicative of CCS, do indeed suffer from CCS. Such is now presented herein: A plasma sample from a patient suspected to suffer from, or being at risk of suffering from, chronic coronary syndrome is fractionated into sub-fractions and the concentration of a single or a set of proteins is determined in said fractions. It is known that the proteins in said fractions are associated with the extracellular vesicles within said fractions. Subsequently, the concentrations are compared to concentrations determined in reference samples from a control human subject and when compared, the determined concentration in said fractions can now tell the physician or GP whether a suspected patient indeed suffers from CCS.
The present invention also relates to a protein marker for the diagnosis of CCS in a human patient, wherein said protein marker is selected from the group consisting of: human CD31, NT-pro-BNP and Cathepsin D; or wherein said protein marker is a combination of at least two of the group consisting of: CD31, NT-pro-BNP and Cathepsin D. It was found that when at least one value of the one or more value of step b) of the method was determined, each of the AUC values derived from the concentration of NT-proBNP in either LDL or HDL plasma fraction, the concentration of CD31 in the LDL plasma fraction, the concentration of Cathepsin D in the LDL plasma fraction or the ratio involving the concentrations of either NT-pro-BNP, CD31 or Cathepsin D in the two plasma fractions individually appeared to be highly indicative. The AUC value increased when combinations of protein marker concentrations and certain sub-fractions were combined. As outlined in the accompanying examples, the inventors found that especially the combinations CatD-HDL + BNP-LDL, CD31-HDL + BNP-LDL, CatD-HDL + BNP-HDL, CD31-HDL + BNP-HDL, CatD-LDL + BNP-LDL, CatD-LDL/HDL +BNP-LDL, CatD-LDL + BNP-HDL, CatD-HDL + CD31-LDL/HDL, CatD-HDL + CD31-LDL, CD31-LDL/HDL + BNP-LDL and CD31-LDL + BNP-HDL were indicative for stable angina. Moreover, the combination of NT-proBNP in LDL with the ratio of NT-proBNP in LDL over HDLincreased the individual statistical significance of the concentration of NT-proBNP in the LDL plasma fraction.
The present invention also relates to a kit for performing the method according to the invention, said kit comprising the means for determining the concentration of said protein markers in said fractions. Said means may comprise any suitable means known in the art, such as an immune-bead based process as outlined herein. Further, the kit may comprise instructions and means for the fractionation of a plasma sample to enable the rapid fractionation and subsequent protein concentration determination as outlined herein.

It should be noted that the above-mentioned embodiments illustrate rather than limit the invention, and that those skilled in the art will be able to design many alternative embodiments.
In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The mere fact that certain measures are recited in mutually different dependent claims does not indicate that a combination of these measures cannot be used to advantage.

### EXAMPLES

### Example 1. Olink Proteomic study on Myomarker stable angina cohort

The MYOMARKER stable coronary artery disease cohort (abbreviation for **MYO**cardial ischemia detection by circulating bio**MARKER**s**)** is a single center, prospective cohort study of patients, who are evaluated in the Meander Medical Center Cardiology outpatient clinic for (recent onset) suspected symptomatic coronary artery disease, undergoing radionuclide myocardial perfusion imaging (rMPI) as indicated by their own cardiologist. Ahead of rMPI, venous blood (6x10cc) is obtained from the peripheral intravenous cannula, which is inserted as part of standard preparation for rMPI. The plasma component is frozen and stored at -80°C within 1 hour after sample collection. All patients are evaluated and managed according to the international guidelines, by their own cardiologist. Patient enrollment started in August 2014 and continued until at least 1250 patients are participating in the study.. Patients younger than 18 years and patients who are unable or unwilling to give their informed consent are not included in this study.

### Data acquisition

Patient information is collected and documented in a digital case record form: clinical presentation, medical history, cardiovascular risk factors, current medication use, ECG evaluation, blood biochemical parameters and results of additional investigations.

### Endpoint adjudication

Objectified ischemic coronary artery disease resulting in CCS or stable angina, as determined by radionuclide myocardial perfusion imaging (Rubidium-82) and adjudicated by a panel of at least two nuclear medicine physicians.

### Follow-up

Patients are followed up for at least 1 year after inclusion. Local hospital records were checked for hospitalization (heart failure or unstable angina), additional cardiac and/or coronary imaging, coronary revascularization, myocardial infarction and death. Patients were contacted by letter and/or telephone and were asked if they had been admitted to a hospital since inclusion. When this was the case, the hospital records were checked for event verification, also when patients were admitted to a center other than the center of inclusion. Major adverse cardiovascular events (MACE) were scored, defined as non-fatal MI (STEMI/NSTEMI type 1), coronary revascularization (PCI or CABG), hospital admission for heart failure or unstable angina and/or all cause death during 1 year of follow-up.

### Preparation of plasma fractions

Extracellular vesicle plasma sub-fraction isolation using sequential precipitation was generally performed as follows. As previously described (Burnstein et al. Rapid method for the isolation of lipoproteins from human serum by precipitation with polyanions. J Lipid Res 1970;11:583-595), Dextran Sulphate (DS) and Manganese (II) chloride (MnCl₂) solution was used to precipitate LDL and the HDL plasma fractions. Briefly, a stock of DS and MnCl₂ were prepared as 6.5% and 2M solutions respectively. For precipitation of the LDL fraction, DS stock (1:125) and MnCl₂ stock (1:40) were added into another 125 µL plasma and mixed. The mixed sample was centrifuged for 10 min at 4,800g at 4ºC precipitating the LDL fraction pellet. This pellet was dissolved in 125 µL lysis buffer and used in the quantitative magnetic bead assays as the LDL fraction. For the HDL fraction, 60 µL of supernatant above the LDL pellet was transferred to a new tube topped up with 65 µL Phosphate-Buffered-Saline (PBS) and mixed. Next, DS stock (1:10) and MnCl₂ stock (1:10) were added into the 125 µl diluted supernatant and mixed. Subsequently, the sample was incubated for 2 h at 4ºC and the sample was centrifuged for 10 min at 4,800g at 4ºC to collect the HDL fraction pellet. This pellet was dissolved in 125 µL Roche lysis buffer and used in the quantitative magnetic bead assays as the HDL fraction.

### Olink Proteomics

### Case control analysis

For this study, a case control analysis of 44 men was performed using the MYOMARKER (MYOcardial ischemia detection by circulating bioMARKERs) study cohort. In short, all patients were >18 year and suspected of CCS at the outpatient clinic of the Meander Medical Center between august 2014 and September 2016. All patients received an ⁸²Rb PET/CT as part of their diagnostic work-up. Cases were defined as patients with stress-induced ischemia based on the adjudication of the presence of the on the results of both myocardial perfusion imaging (MPI), and coronary angiography (CAG) data if available. Rubidium-82 PET/CT MPI results were assessed according to the 17-segment model of the American Heart Association. All scans were evaluated by 2 experienced observers. In short, the summed difference score (SDS) was the total difference between the stress and rest score for each of the 17 segments. Cases (patients with stable IHD) were defined as patients with SDS score ≥ 2 and visual agreement by both observers. Patients were considered as control if their SDS score was <2. CAG images were interpreted with quantitative coronary angiography (QCA) by 2 experienced clinicians using Cardiovascular Angiography Analysis System software (CAAS 7.3, Pie Medical Imaging, Maastricht, The Netherlands). Controls were matched based on age and general cardiovascular risk factors.

Baseline characteristics of the 44 patients can be found in table 1. Mean age of the study population was 63 years in the control group and 68 in the cases (NS). As can be seen, no differences were found regarding traditional risk factor for CCS. Approximately 50% in both groups was already known with coronary artery disease.

**Table 1. Baseline characteristics**

| | Control | Case | P value |
|---|---|---|---|
| n | 22 | 22 | |
| Age | 63.32 (10.27) | 67.86 (16.23) | 0.273 |
| BMI | 29.79 (4.20) | 27.97 (4.31) | 0.174 |

| **Risk factors** | | | |
|---|---|---|---|
| Smoking | 5 (22.7) | 2 (9.1) | 0.410 |
| Diabetes | 6 (27.3) | 6 (27.3) | 1.000 |
| Hypertension | 12 (54.5) | 14 (63.6) | 0.759 |
| Hypercholesterolemia | 12 (54.5) | 12 (54.5) | 1.000 |
| Family history of CAD | 8 (36.4) | 10 (45.5) | 0.759 |

| **Medical history** | | | |
|---|---|---|---|
| Cardiovascular disease | 16 (72.7) | 19 (86.4) | 0.455 |
| Coronary artery disease Coronary | 9 (40.9) | 12 (54.5) | 0.546 |
| revascularization | 8 (36.4) | 10 (45.5) | 0.759 |

| **Medication** | | | |
|---|---|---|---|
| Anti hypertensiva | 15 (68.2) | 18(81.8) | 0.486 |
| Lipidlowering | 13 (59.1) | 14 (63.6) | 1.000 |
| Anticoagulans | 2 (9.1) | 5 (22.7) | 0.410 |
| Antiplatelet | 11 (50.0) | 13 (59.1) | 0.762 |

| | | | |
|---|---|---|---|
| Values are shown as mean (SD) or number with corresponding frequency. Case is defined as stress-induced ischemia objected with myocardial perfusion imaging. CAD = Coronary Artery Disease. | | | |

### Olink

Biomarker analysis was performed with the commercially available Proximity Extension Assay (PEA) technique (Olink Proteomics, Uppsala, Sweden). Two panels (CVD III PEA and Cardiometabolics PEA) were selected based on their presumed role within CVD. Both panels included 92 proteins and were measured in all 44 men and in both plasma EV subfractions (e.g. LDL and HDL). The technique is based on the binding of oligonucleotide-conjugated antibodies, their binding to their epitopes on the target protein form a sequence to target with quantitative real time PCR (qRT-PCR). The qRT-PCR data were transformed into NPX values (normalized protein expression).
Proteins selected for verification was done based on their diagnostic properties (sensitivity, specificity, negative predictive value and positive predictive value), their presumed role in CVD based on literature and the availability of antibodies and assays.

As a result, the inventors found the following proteins to be of particular relevance: NT-proBNP derived from the full length *UniProtKB* - *P16860 (ANFB_HUMAN)* protein; CD31 (also known as PECAM-1) *UniProtKB* - *P16284 (PECA1_HUMAN);*
Cathepsin D *UniProtKB* - *P07339 (CATD_HUMAN).*

### Example 2. Verification of extracellular vesicle proteins in plasma subfractions for diagnosis of stable angina

Plasma subfractions were prepared as described under Example 1 using the same case control cohort as shown under example 1 (Table 1) The protein levels in each subfraction were measured by electrochemiluminescence immunoassay (Quickplex SQ120, Meso Scale) and original essay units were expressed as pg/mL.

Figure 1A-C shows boxplots with MSD results for the selected proteins (Cathepsin D, CD31 and NT-proBNP) in the HDL subfraction. In figure 1D-F the results for the LDL subfraction are summarized. In the HDL subfraction only NT-proBNP was found to be different between cases and controls when taken as marker alone.

### Example 3. Statistical Analyses for Stable Angina (Myomarker cohort)

For this, the 3 extracellular vesicle proteins Nt-proBNP, CTSD, CD31 were measured in the plasma fractions LDL and HDL in the cohort of 22 stable coronary artery disease patients of the Myomarker cohort and 22 sex-, age, history-, and medication-matched controls of the same cohort using sequential precipitation as described under example 1 above and an MSD assay as described under example 2 above. The best performing individual marker/fraction pairs and marker/ratio pairs for identification of stable angina are listed in tables 2 and 3. Table 2 lists all marker/fraction pairs and marker/ratio pairs that have been used to compare chest-pain patients diagnosed with CCS using perfusion imaging (Case) and chest-pain patients that do not have CSS (control). The p-value is based on the biomarker values comparison between case and control. Table 3 lists the most significant and best performing marker/fraction pairs and marker/ratio pairs, which on their own, and for combination with further marker/fraction pairs or marker/ratio pairs, provide a statistically relevant (p<0.05) identification of subjects suffering from stable angina. Table 3 shows the AUC values obtained in the ROC plots of these most significant and best performing individual marker/fraction pairs or marker/ratio pairs and for combination with further marker/fraction pairs or marker/ratio pairs. The ROC plots are performed on the data from the above-mentioned patient and control groups of the Myomarker cohort..

**Tabel 2. Comparison between chest-pain patients diagnosed with CCS using perfusion imaging (Case) and chest-pain patients that do not have CSS for the 3 selected markers and combinations of markers in the two plasma fractions LDL and HDL. p-value is based on the biomarker values comparison between case and control. BNP = NT-proBNP; CATD or CatD = Cathepsin D.**

| **Biomarker** | **Control** | **Case** | ***p* value** |
|---|---|---|---|
| n | 22 | 22 | |
| BNP_LDL | 5.00 [2.98, 8.88] | 21.62 [8.18, 64.72] | <0.001 |
| BNP_HDL | 2.30 [0.97, 4.39] | 6.06 [3.18, 15.27] | 0.004 |
| CATD_LDL | 4909 [3706, 5824] | 6160 [4903, 7300] | 0.02 |
| CD31_LDL | 2756 [2340, 3753] | 4273 [2975, 6251] | 0.031 |
| CD31_HDL | 1217 [348, 1694] | 616 [423, 956] | 0.197 |
| CATD_HDL | 1219 [756, 1675] | 983 [771, 1290] | 0.411 |

| **Biomarker ratio & combinations** | | | |
|---|---|---|---|
| CatDHDL/BNPLDL | 205 [128.96, 610.45] | 32 [21, 112] | <0.001 |
| CD31HDL/BNPLDL | 160 [103, 304] | 27 [13, 54] | <0.001 |
| CatDLDL/BNPLDL | 1207 [510.82, 1706.26] | 264 [114, 659] | 0.001 |
| CatDHDL/BNPHDL | 610 [303.31, 973.78] | 126 [62, 355] | 0.001 |
| CD31HDL/BNPHDL | 357 [199, 838] | 91 [44, 217] | 0.001 |
| BNPLDL/BNPLDLHDL | 2.30 [0.97, 4.39] | 6.06 [3.18, 15.27] | 0.004 |
| BNPLDLHDL | 2.37 [1.77, 3.49] | 3.49 [2.78, 4.58] | 0.006 |
| CD31LDL/BNPLDL | 602 [293, 922] | 163 [62, 575] | 0.006 |
| CD31LDLHDL | 2.74 [2.01, 5.74] | 5.75 [4.35, 10.86] | 0.01 |
| CatDLDLHDL/BNPLDL | 0.74 [0.40, 2.20] | 0.30 [0.09, 0.84] | 0.01 |
| CatDLDLHDL | 4.19 [3.22, 5.85] | 6.17 [5.08, 7.89] | 0.015 |
| CatDLDL/BNPHDL | 2466 [1199.63, 4743.18] | 1104 [330, 2152] | 0.023 |
| CatDHDL/CD31LDLHDL | 334 [127.44, 871.97] | 139 [96, 220] | 0.035 |
| CatDHDL/CD31LDL | 0.44 [0.20, 0.83] | 0.23 [0.16, 0.36] | 0.044 |
| CD31LDLHDL/BNPLDL | 0.70 [0.31, 2.68] | 0.26 [0.08, 0.89] | 0.054 |
| CatDLDL/CD31LDLHDL | 1592 [824, 2773] | 1003 [495, 1312] | 0.057 |
| CD31LDL/BNPHDL | 1477 [802, 3516] | 643 [258, 1827] | 0.057 |
| CD31HDL/CD31LDLHDL | 469 [70, 994] | 130 [47, 187] | 0.06 |
| CatDHDL/BNPLDLHDL | 562 [196.79, 830.43] | 267 [206, 380] | 0.064 |
| CatDLDLHDL/BNPHDL | 1.61 [0.76, 5.67] | 1.08 [0.35, 2.38] | 0.071 |
| BNPHDL/BNPLDLHDL | 1.06 [0.36, 2.36] | 1.67 [0.93, 5.39] | 0.078 |
| CatDLDL/CD31HDL | 4.79 [3.29, 12.25] | 8.25 [6.45, 12.51] | 0.082 |
| CatDHDL/CatDLDLHDL | 300 [138.54, 566.89] | 137 [109, 248] | 0.082 |
| CD31LDLHDL/BNPLDLHDL | 1.20 [0.91, 2.09] | 1.89 [1.24, 2.71] | 0.105 |
| CD31HDL/BNPLDLHDL | 598 [94, 806] | 176 [90, 311] | 0.11 |
| CatDLDLHDL/CD31LDLHDL | 1.38 [0.85, 2.00] | 1.17 [0.60, 1.55] | 0.146 |
| CatDLDLHDL/CD31HDL | 0.00 [0.00, 0.02] | 0.01 [0.01, 0.02] | 0.159 |
| CD31LDL/CD31LDLHDL | 1217 [347, 1693] | 616 [424, 956] | 0.197 |
| CD31LDLHDL/BNPHDL | 2.10 [0.45, 5.99] | 1.09 [0.31, 3.20] | 0.241 |
| CatDLDL/BNPLDLHDL | 2264 [1274.39, 2862.19] | 1820 [1292, 2039] | 0.28 |
| CatDLDL/CD31LDL | 1.85 [1.15, 2.40] | 1.29 [1.14, 2.06] | 0.302 |
| CatDLDL/CatDLDLHDL | 1219 [756, 1675] | 983 [771, 1290] | 0.411 |
| CatDLDLHDL/CD31LDL | 0.00 [0.00, 0.00] | 0.00 [0.00, 0.00] | 0.814 |
| CatDLDLHDL/BNPLDLHDL | 1.75 [1.56, 1.87] | 1.83 [1.48, 1.98] | 0.851 |
| CD31LDL/BNPLDLHDL | 1353 [664, 1882] | 1336 [900, 1623] | 0.851 |
| CatDHDL/CD31HDL | 1.30 [0.94, 2.32] | 1.22 [1.07, 2.29] | 0.963 |

**Tabel 3. Area under Curve (AUC) with Upper and Lower range showing significance compared to AUC of 0,5: If 0.5 falls between Upper and Lower range the AUC is not significantly different from AUC 0.5. This significance compared to AUC 0.5 is indicated with NS (Not significant) and p<0.05 (indicating significant). BNP = NT-proBNP; CATD or CatD = Cathepsin D**

| **Biomarker** | **AUC** | **AUC-lower** | **AUC-upper** | **Significance** |
|---|---|---|---|---|
| CATD_HDL | 0,572 | 0,395 | 0,75 | NS |
| CD31_HDL | 0,614 | 0,434 | 0,793 | NS |
| CD31_LDL | 0,69 | 0,528 | 0,852 | p<0.05 |
| CATD_LDL | 0,705 | 0,545 | 0,864 | p<0.05 |
| BNP_HDL | 0,756 | 0,612 | 0,9 | p<0.05 |
| BNP_LDL | 0,833 | 0,708 | 0,958 | p<0.05 |

| **Biomarker ratios & combinations** | | | | |
|---|---|---|---|---|
| CatDHDL/CD31HDL | 0,504 | 0,327 | 0,682 | NS |
| CatDLDLHDL/BNPLDLHDL | 0,517 | 0,338 | 0,695 | NS |
| CD31LDL/BNPLDLHDL | 0,517 | 0,34 | 0,693 | NS |
| CatDLDLHDL/CD31LDL | 0,521 | 0,343 | 0,699 | NS |
| CatDLDL/CatDLDLHDL | 0,572 | 0,395 | 0,75 | NS |
| CatDLDL/CD31LDL | 0,591 | 0,417 | 0,764 | NS |
| CatDLDL/BNPLDLHDL | 0,595 | 0,415 | 0,775 | NS |
| CD31LDLHDL/BNPHDL | 0,603 | 0,432 | 0,774 | NS |
| CD31LDL/CD31LDLHDL | 0,614 | 0,434 | 0,793 | NS |
| CatDLDLHDL/CD31HDL | 0,624 | 0,442 | 0,806 | NS |
| CatDLDLHDL/CD31LDLHDL | 0,628 | 0,457 | 0,799 | NS |
| CD31HDL/BNPLDLHDL | 0,64 | 0,462 | 0,819 | NS |
| CD31LDLHDL/BNPLDLHDL | 0,643 | 0,47 | 0,816 | NS |
| CatDLDL/CD31HDL | 0,653 | 0,479 | 0,826 | NS |
| CatDHDL/CatDLDLHDL | 0,653 | 0,483 | 0,823 | NS |
| BNPHDL/BNPLDLHDL | 0,655 | 0,49 | 0,82 | NS |
| CatDLDLHDL/BNPHDL | 0,659 | 0,497 | 0,821 | NS |
| CatDHDL/BNPLDLHDL | 0,663 | 0,491 | 0,836 | NS |
| CD31HDL/CD31LDLHDL | 0,665 | 0,49 | 0,841 | NS |
| CatDLDL/CD31LDLHDL | 0,667 | 0,498 | 0,836 | NS |
| CD31LDL/BNPHDL | 0,667 | 0,503 | 0,832 | p<0.05 |
| CD31LDLHDL/BNPLDL | 0,669 | 0,507 | 0,832 | p<0.05 |
| CatDHDL/CD31LDL | 0,678 | 0,513 | 0,842 | p<0.05 |
| CatDHDL/CD31LDLHDL | 0,686 | 0,518 | 0,854 | p<0.05 |
| CatDLDL/BNPHDL | 0,7 | 0,543 | 0,858 | p<0.05 |
| CatDLDLHDL | 0,715 | 0,552 | 0,878 | p<0.05 |
| CD31LDLHDL | 0,727 | 0,566 | 0,888 | p<0.05 |
| CatDLDLHDL/BNPLDL | 0,727 | 0,576 | 0,879 | p<0.05 |
| CD31LDL/BNPLDL | 0,74 | 0,586 | 0,894 | p<0.05 |
| BNPLDLHDL | 0,744 | 0,594 | 0,894 | p<0.05 |
| BNPLDL/BNPLDLHDL | 0,756 | 0,612 | 0,9 | p<0.05 |
| CatDLDL/BNPLDL | 0,783 | 0,646 | 0,921 | p<0.05 |
| CD31HDL/BNPHDL | 0,8 | 0,665 | 0,934 | p<0.05 |
| CatDHDL/BNPHDL | 0,804 | 0,669 | 0,939 | p<0.05 |
| CD31HDL/BNPLDL | 0,845 | 0,725 | 0,965 | p<0.05 |
| CatDHDL/BNPLDL | 0,847 | 0,728 | 0,966 | p<0.05 |

The p-values, negative predictive values and positive predictive values between case and control were found to be the same for the ratio of two values derived from the concentration of a single marker protein in the respective fractions, irrespective whether the ratio was determined from the concentration of the marker in the LDL fraction over that in the HDL fraction or from the concentration of the marker in the HDL fraction over that in the LDL fraction. For example, both marker/ratio pairs CatD-LDLHDL and CatD-HDLLDL have a similar p-value of 0.015, a similar AUC of 0.715 with similar AUC-upper- and -lower values, similar negative predictive value of (NPV) of 83.3% and a similar positive predictive value (PPV) of 73.1%. Hence, the values resulting from a ratio involving LDL/HDL listed in tables 2 and 3 apply to the inverse ratio of HDL/LDL as well.

The three best marker combinations appeared to be 1) Cathepsin D in the HDL fraction (CatDHDL) with NT-proBNP in LDL (BNPLDL) and an AUC of 0.847 (95% CI 0.728-0.966); 2) CD31 in HDL and BNPLDL with an AUC of 0.845 (95% CI 0.725-0.965)) and 3) BNPLDL with an AUC of 0.833 (95% CI 0.708-0.958) to discriminate between CCS and matched controls. Selection of the best combination of markers and sub-fractions was done using Logistics regression analysis with forward selection based upon Akaike Information Criterion (AIC). These results show that using the same set of extracellular protein levels in two fractions can be used for stable angina. Figure 2 shows the AUC curves of the 3 highest scoring combinations of marker/sub-fraction for the Myomarker cohort.

## Claims

1. Method for identifying a subject suffering from, or being at risk of suffering from chronic coronary syndrome, said method comprising the steps of:
a) obtaining one or more of the plasma fractions selected from the group consisting of the Low-Density Lipoprotein (LDL) fraction and the High-Density Lipoprotein (HDL) fraction from a plasma sample of said subject;
b) determining one or more values, wherein each of the one or more values:
- is derived from a concentration of a protein marker in one of said plasma fractions, wherein the protein marker is selected from the group consisting of CD31, NT-proBNP and/or Cathepsin D, and/or
- is a ratio of two values derived from the concentrations of a single protein marker in two different ones of said plasma fractions, wherein the protein marker is selected from the group consisting of CD31, NT-proBNP and/or Cathepsin D,
c) performing a comparison of the one or more values as determined in step b) with one or more corresponding reference values, which has been derived in the same way from the concentration of the same one or more protein marker in corresponding plasma fractions as determined in group of reference subjects not suffering from chronic coronary syndrome, wherein a statistically significant difference between the one or more values determined in step b) and the one or more corresponding reference values is indicative of the subject suffering, or being at risk of suffering, from chronic coronary syndrome.

2. Method according to claim 1, wherein the one or more values in step b) are selected such that the area under the curve (AUC) differs from the diagonal reference line of 0.5 with a p-value of 0.05 or less, as determined by Receiver Operating Characteristic (ROC) plot analysis of the combination of said one or more values based on a suitable group of definitive subjects and group of reference subjects.

3. Method according to claim 1, wherein the one or more values in step b) are selected such that the area under the curve (AUC) is 0.8 or more and the p-value is 0.05 or less, as determined by Receiver Operating Characteristic (ROC) plot analysis of the combination of said one or more values based on a suitable group of definitive subjects and group of reference subjects.

4. Method according to any of the preceding claims, wherein the negative predictive value and/or the positive predictive value is 0.8 or more.

5. Method according to any of the preceding claims, wherein at least one of the one or more values is selected from the group consisting of:
- a value derived from the concentration of CD31 in the LDL plasma fraction,
- a value derived from the concentration of NT-proBNP in the LDL plasma fraction,
- a value derived from the concentration of CTSD in the LDL plasma fraction, and
- a value derived from the concentration of NT-proBNP in the HDL plasma fraction;
and/or wherein at least one of the one or more values is selected from the group consisting of:
- the ratio of a value derived from the concentration of CD31 in the LDL plasma fraction over a value derived from the concentration in the HDL plasma fraction,
- the ratio of a value derived from the concentration of NT-proBNP in the LDL plasma fraction over a value derived from the concentration in the HDL plasma fraction, and
- the ratio of a value derived from the concentration of CTSD in the LDL plasma fraction over a value derived from the concentration in the HDL plasma fraction.

6. Method according to any of the preceding claims, wherein step b) comprises determining at least two values.

7. Method according to claim 6, wherein step b) comprises determining at least two values involving two different protein markers selected from the group of CD31, NT-proBNP and/or Cathepsin D.

8. Method according to any of the preceding claims wherein at least one value involves the protein marker NT-proBNP.

9. Method according to claim 7 or 8, wherein the at least two values involving two different protein markers are selected from the group consisting of:
- the combination of a value derived from the concentration of Cathepsin D in the HDL plasma fraction and a value derived from the concentration NT-proBNP in the LDL plasma fraction;
- the combination of a value derived from the concentration of CD31 in the HDL plasma fraction and a value derived from the concentration NT-proBNP in the LDL plasma fraction;
- the combination of a value derived from the concentration of Cathepsin D in the HDL plasma fraction and a value derived from the concentration NT-proBNP in the HDL plasma fraction;
- the combination of a value derived from the concentration of CD31 in the HDL plasma fraction and a value derived from the concentration NT-proBNP in the HDL plasma fraction; and
- the combination of a value derived from the concentration of Cathepsin D in the LDL plasma fraction and a value derived from the concentration NT-proBNP in the LDL plasma fraction.

10. Kit comprising detecting means for performing the method defined in any of the preceding claims.
